# EUROPEAN PATENT SPECIFICATION

(11) **EP 4 293 568 B1**
(45) Date of publication and mention of the grant of the patent: **27.08.2025**
(21) Application number: 23207691.9
(22) Date of filing: 23.01.2019
(51) Int. Cl.: G01N 33/543, G01N 33/53, G06K 19/06

(54) **MICROBEAD**
MIKROPERLE
MICROBILLE

(43) Date of publication of application: 20.12.2023
(62) Divisional of application: 19911678.1
(73) Proprietor: BGI Shenzhen, Shenzhen, Guangdong 518083 (CN)
(72) Inventor: ZHAN, Wenwei, 518083 Shenzhen (CN); CHEN, Wei, 518083 Shenzhen (CN); WANG, Weimao, 518083 Shenzhen (CN); LI, Mei, 518083 Shenzhen (CN); LI, Yuxiang, 518083 Shenzhen (CN); WANG, Jian, 518083 Shenzhen (CN)
(74) Representative: Zaboliene, Reda

(56) References cited:
- WO-A1-2016/198954
- WO-A1-2018/052464

## Description

### FIELD

The subject matter relates to biological detection, and more particularly, to a microbead used in the biological detection.

### BACKGROUND

Multiple assays are very important in immunological assays and molecular diagnostic assays. The multiple assay refers to simultaneous quantifying multiple analytes in a single assay. The multiple assay uses multiple capture agents, and each capture agent being specific with a target macromolecule. In a chip-based multiplex assay, each type of capture agent is attached to a predetermined location of the chip. The amounts of multiple targets in a complex sample are determined by detecting signals of the molecules at each location of the same type of capture agent. In suspension array multiplex assay, microbeads are suspended in the solution. These microbeads contain identification elements (such as "codes") that can be embedded, printed, or produced by one or more elements of the microbeads. Each type of capture agent is fixed to the microbeads with the same code, and the signals emitted from the detected molecules on the microbeads with the specific code reflect the amount of the corresponding targets.

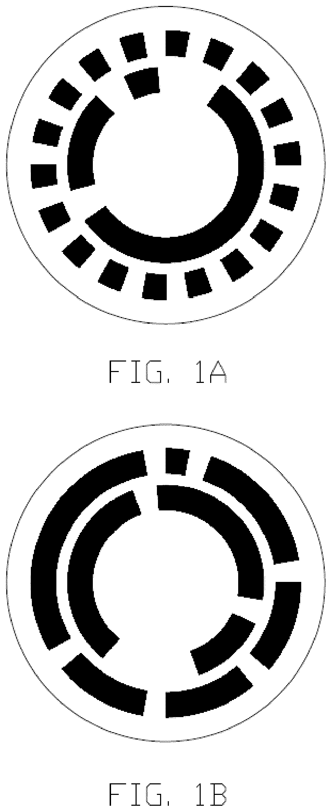

The current codes of the microbead are produced by marking an opaque substance or fluorescent substance inside the microbead. The codes of the microbead can be identified by a distribution of the opaque substance or fluorescent substance. As shown in FIGS. 1A and 1B, the black region represents the opaque substance or fluorescent substance. However, such coding method has following shortcomings. 1) The production cost of the microbead is high, and additional processes and materials are needed to mark the codes. 2) There are several subsequent identification steps, and the identification of such codes needs additional white light image or specific fluorescence image of the microbead. 3) Forming the codes inside the microbead limits the number of codes and the size of the region for biological reaction.

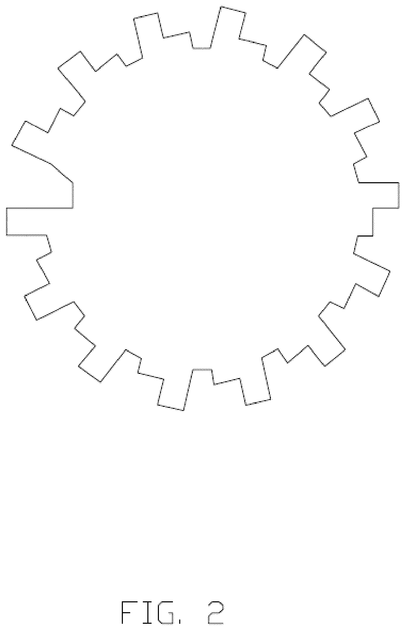

There is another way in this field for coding microbead, by engraving codes of different depths, different shapes, and different intervals around the circular microbeads, thereby greatly increasing the number of coding combinations as shown in FIG. 2. However, such coding method also has following shortcomings. 1) The microbead is round, and fewer codes can be engraved on per unit region of the microbead. 2) The positioning of the microbead is difficult. During image processing, it is difficult to positioning the microbead, and the positioning accuracy is low. 3) There is a gap between the codes, which wastes space and reduces the length of the codes. 4) The direction of the codes can only be obtained by the position of the mark. When the mark position is shielded, damaged, or the identification thereof is wrong, the direction of the codes may be unidentified or even wrongly identified. Conventional microbeads are known from D1 (WO 2018/052464A1) and D2 (WO 2016/198954A1).

### SUMMARY

Therefore, a microbead that can overcome at least one of the above shortcomings. The invention is set out in the appended set of claims.

The present disclosure provides a microbead with a code engraved on outside of the microbead. The microbead includes a central region and an edge region surrounding the central region. An outer contour of the edge region before and after engraving the code is non-circular. The edge region includes a plurality of coding positions, the code of the microbead is engraved on the plurality of coding positions, and each bit of the code corresponds to each of the plurality of coding positions. The edge region includes corner regions and side regions, and the corner regions and the side regions are connected to each other in a direction surrounding the central region. The corner regions are provided with positioning devices and/or marking positions, and the positioning devices and/or the marking positions are configured to allow a computer identification device to identify a front side and a back side of the microbead, a starting position of the code, and a direction of the code.

In one embodiment, the microbead has a first length in a first dimensional direction and a second length in a second dimensional direction perpendicular to the first dimensional direction. The first length is longer than the second length, and the edge region surrounds the central region at least on a plane formed by the first dimensional direction and the second dimensional direction.

In one embodiment, the plurality of coding positions is at least arranged on the plane formed by the first dimensional direction and the second dimensional direction.

In one embodiment, a portion of the corner regions is processed to have a shape different from other portions of the corner regions, and the portion of the corner region as a whole serves as the positioning device.

In one embodiment, a portion of the corner regions defines a through hole penetrating the microbead, and the through hole serves as the positioning device. Or a portion of the corner regions is provided with a positioning protrusion, and the positioning protrusion serves as the positioning device.

In one embodiment, the marking position is an oblique line segment or an arc segment generated by cutting one of the corner regions.

In one embodiment, a center of a circle where the arc segment is located is on the microbead.

In one embodiment, the plurality of coding positions is disposed on the side regions. Or the plurality of coding positions is disposed on the side regions and the corner regions.

In one embodiment, the code is presented by engraving code patterns on the plurality of coding positions, and the code patterns is a combination of one or more patterns.

In one embodiment, the coding patterns are triangular coding patterns, rectangular coding patterns, trapezoidal coding patterns, or any combination thereof.

In one embodiment, the coding pattern of at least one of the plurality of coding positions is used to indicate a front side and a back side of the microbead and a direction of the code.

In one embodiment, the coding pattern of the at least one of the plurality of coding positions has a preset vertex, the preset vertex deviates to or from a preset direction, and the preset direction is the direction of the code on the microbead.

In one embodiment, at least one of the coding patterns has directionality, and the directionality of the coding pattern is used as a constituent factor of the code.

In one embodiment, the coding pattern having the directionality further has a preset vertex. The preset vertex deviates from or to one of at least two different preset directions, and the different preset directions which the preset vertex deviates from or to represent another different code.

In the microbead of the present disclosure, since the shape of the microbead deviates from circular, compared to the circular microbead, a ratio of the overall surface area of the non-circular microbead with respect to the perimeter of the microbead before coding is smaller, and more coding positions can be set on the same consumable region, or the size of the consumable region can be reduced by setting a same number of coding positions. Thus, the utilization rate of microbeads can be improved.

### BRIEF DESCRIPTION OF THE DRAWINGS

Implementations of the present technology will now be described, by way of embodiment, with reference to the attached figures. Obviously, the drawings are only some embodiments of the present disclosure. For those of ordinary skill in the art, other drawings can be obtained based on these drawings without creative work.
FIGS. 1A and 1B are diagrammatic views wherein codes are within the existing microbead.
FIG. 2 is a diagrammatic view wherein the codes are outside the existing microbead.
FIGS. 3A and 3B are diagrammatic views of the microbead before and after coding according to a first embodiment of the present disclosure.
FIGS. 4A and 4B are diagrammatic views of the microbead before and after coding according to a second embodiment of the present disclosure.
FIGS. 5A and 5B are diagrammatic views of the microbead before and after coding according to a third embodiment of the present disclosure.
FIGS. 6A and 6B are diagrammatic views of the microbead after coding according to a fourth embodiment of the present disclosure.
FIGS. 7A and 7B are diagrammatic views of the microbead before and after coding according to a fifth embodiment of the present disclosure.
FIGS. 8A and 8B are diagrammatic views of the microbead before and after coding according to a sixth embodiment of the present disclosure.
FIGS. 9A and 9B are diagrammatic views of the microbead before and after coding according to a seventh embodiment of the present disclosure.
FIGS. 10A and 10B are diagrammatic views of the microbead before and after coding according to an eighth embodiment of the present disclosure which is not part of the invention.
FIG. 11 is a diagrammatic view of the microbead after coding according to a ninth embodiment of the present disclosure which is not part of the invention.
FIG. 12 is a block diagram of a computer identification device for identifying the code of the microbead according to the embodiment of the present disclosure.

### Symbol description of main components

Microbead 10, 20, 30, 40a, 40b, 50, 60, 70, 80, 90, 1000
Edge region 11, 51, 81
Central region 12, 52, 82
Corner region 13, 13a, 13b, 23a, 33a, 33b, 43a, 53, 53a, 53b, 63a, 63b, 73a, 73b
Side region 14, 54
Marking position A
Coding position 141, 241, 341, 441, 541, 641, 731, 741, 831, 841
Positioning device 631a
Straight side region 83
Arc side region 84
Vertex 910
Projection h
Midpoint position i
Geometric center X, O
Computer identification device 900
Image capturing module 901
Code identification module 902

### DETAILED DESCRIPTION

Implementations of the disclosure will now be described, by way of embodiments only, with reference to the drawings. The described embodiments are only portions of the embodiments of the present disclosure, rather than all the embodiments. The disclosure is illustrative only, and changes may be made in the detail within the principles of the present disclosure. It will, therefore, be appreciated that the embodiments may be modified within the scope of the claims.

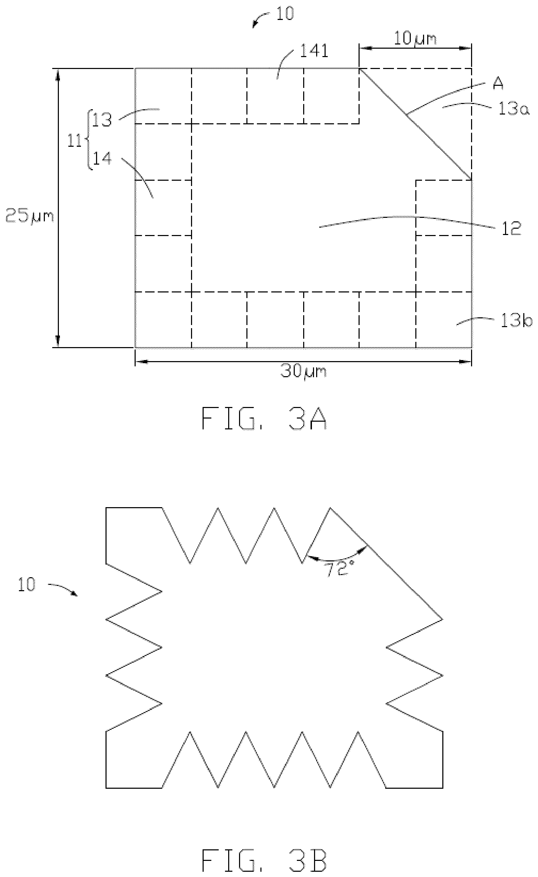

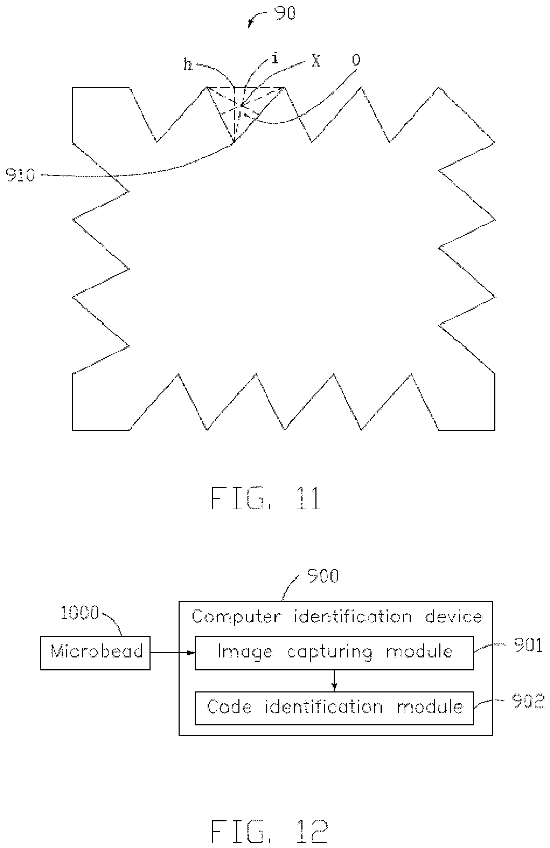

Referring to FIG. 3A and FIG. 3B, which are diagrammatic views of a microbead 10 before and after coding according to a first embodiment of the present disclosure. The microbead 10 is non-circular, which can be rectangular (including square, rectangular), trapezoidal, elliptical, and other regular or irregular shape. Specifically, in the embodiment, the microbead 10 is a rectangular microbead, and the code is engraved on the outside of the microbead 10. Furthermore, the microbead 10 includes an edge region 11 and a central region 12 surrounded by the edge region 11. The edge region 11 includes corner regions 13 and side regions 14 besides the corner region 13. The corner regions 13 are where four corners of the rectangle are located. One corner region 13a includes a marking position A. The remaining three corner regions 13b are used as positioning devices for positioning. The marking position A and the three corner regions 13b for positioning enable a computer identification device 900 (shown in FIG. 12) to accurately position the microbead 10. The side regions 14 are where the long and short sides of the rectangle adjacent to the corner regions 13 are located. At least one coding position 141 is disposed on each side region 14. Each coding position 141 is used to engrave a code pattern. In the embodiment, the marking portion A is an oblique line segment generated by cutting the corner region 13a. Lengths of the long and short sides after cutting the corner region 13 are different. In the embodiment shown in FIG. 3A, for a rectangular microbead 10 having a length of 30 µm, a width of 25 µm, and a consumable region of 750 µm², one corner region 13a of 10*10 µm is cut to form the marking position A. An included angle E is formed between the marking position A and a triangular coding pattern on the adjacent coding position 141, which is approximately 72°. Starting from the mark position A, two adjacent coding positions 141 are set on the first short side next to the mark position A in a clockwise direction. Four adjacent coding positions 141 are set on the first long side next to the first short side in the clockwise direction. Three coding positions 141 are set on the second short side next to the first long side in the clockwise direction. Three coding positions 141 are set on the second long side next to the second short side in the clockwise direction. Thus, a code with a length of 12-bit can be set on the microbead 10, and multiple coding positions on the same side are closely adjacent to each other. In the embodiment shown in FIG. 3B, starting from the marking position A, each coding position 141 is engraved with a coding pattern, and the coding pattern is a triangle with a vertex facing the inside of the microbead 10. In other embodiments, the coding pattern may also be a triangle with the vertex facing the outside of the microbead 10. By engraving the triangular coding patterns on different coding positions 141 to form different coding combinations, 2¹²=4096 different codes can be formed.

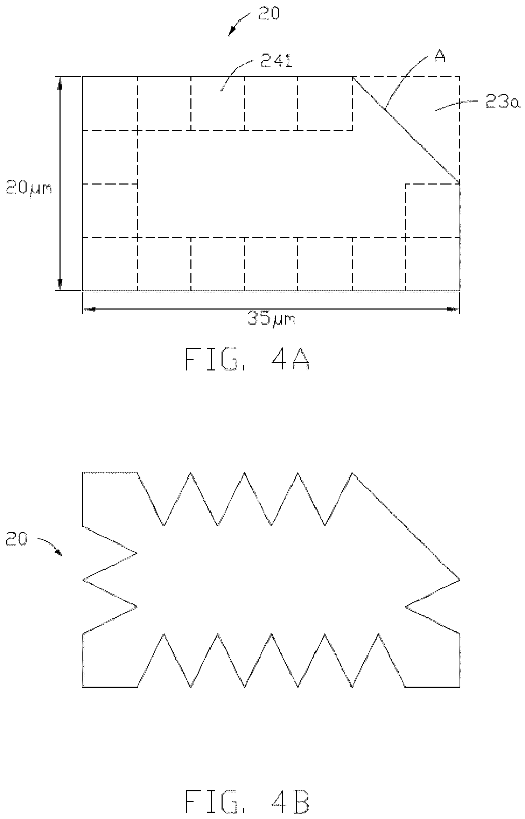

Referring to FIG. 4A and FIG. 4B, which are diagrammatic views of a microbead 20 before and after coding according to a second embodiment of the present disclosure. The microbead 20 in the embodiment is substantially the same as the microbead 10 in the first embodiment, except that the microbead 20 as a whole is more slender than the microbead 10. Specifically, in the embodiment, the long side of the rectangular microbead 20 is 35µm, the short side is 20µm, and the consumable region is 700µm². One corner region 23a of 10*10µm is cut to form the marking position A. Starting from the marking position A, one coding position 241, five coding positions 241, two coding positions 241, and four coding positions 241 are respectively set on the first short side next to the mark position A, the first long side next to the first short side, the second short side next to the first long side, and the second long side next to the second short side. Compared with microbead 10, the microbead 20 has a larger aspect ratio (that is, the ratio of the long side to the wide side), and the code with a length of 12-bit can still be set although the size of consumable region is reduced. FIG. 4B shows one code of the microbead 20. It can be understood that by engraving the triangular code patterns on different code positions 241 to form different code combinations, 2¹²=4096 different codes can be formed.

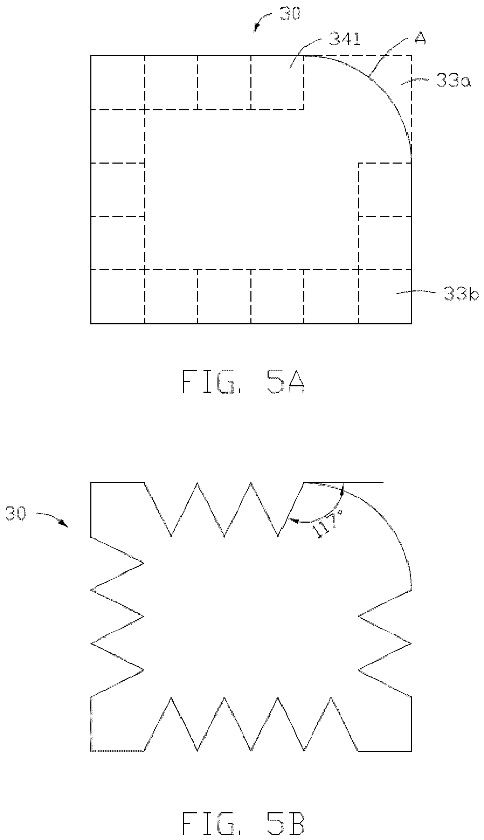

Referring to FIGS. 5A and 5B, which are diagrammatic views of a microbead 30 before and after coding according to a third embodiment of the present disclosure. The microbead 30 in the embodiment is substantially the same as the microbead 10 in the first embodiment, except that the marking position A is an arc segment generated by cutting one corner region 33a. A center of a circle where the arc segment is located is at the microbead 30, and the circle has a radius of 10 µm. The arc segment and the triangular code pattern on the adjacent coding position 341 form an angle of approximately 117°. Therefore, compared with microbead 10 in which the marking position A and the triangular coding pattern on the adjacent coding position 131 forming an included angle of 72°, the marking position A of the microbead 30 is more easily recognized by the computer identification device 900. In addition, the marking position A of the microbead 30 with the arc shape is also more easily recognized by the computer identification device 900. Therefore, the marking position A of the microbead 30 is more easily distinguished by the computer identification device 900 from other corner regions 33b used for positioning, and is more easily recognized by the computer identification device 900. FIG. 5B shows one code of the microbead 30. It can be understood that different code combinations are formed by engraving triangular code patterns on different code positions 341, thereby forming different codes.

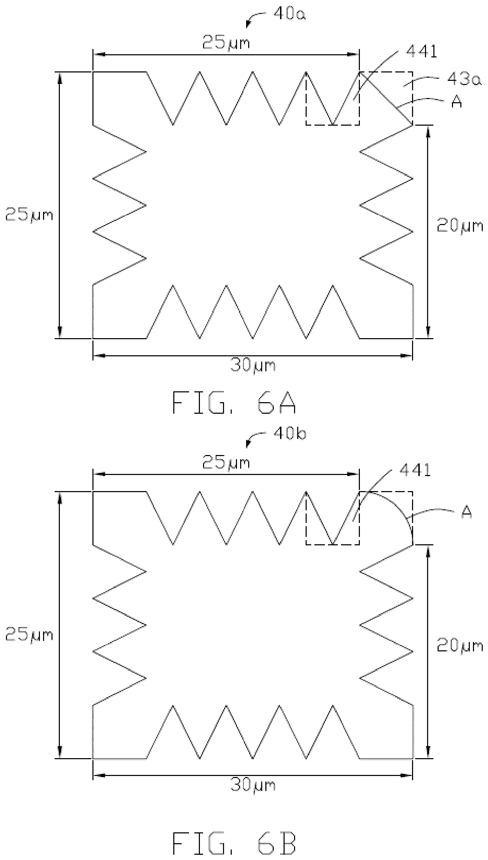

Referring to FIGS. 6A and 6B, which are diagrammatic views of a microbead 40 after coding according to a fourth embodiment of the present disclosure. The microbeads 40a and 40b in the embodiment are substantially the same as the microbeads 10 of the first embodiment and the microbead 30 of the third embodiment, respectively, except that the size of the marking position A is further reduced in the fourth embodiment. Since the size of the marking position A is reduced, the number of coding positions 441 can be increased, thereby increasing the length of the code. The marking position A of the microbead 40a is formed by cutting the corner region 43a of 5*5 µm, and the radius of the circle where the marking position A of the microbead 40b is located is 5 µm. It can be understood that both FIGS. 6A and 6B only show one code of the microbeads 40a and 40b. By engraving the triangular code patterns at different code positions 441, other codes different from those shown in FIGS. 6A and 6B can also be formed.

It can be understood that in other embodiments, the above triangular coding patterns can also be replaced with other types of coding patterns. For example, they can be replaced with rectangular patterns, trapezoidal patterns, etc., or a combination thereof.

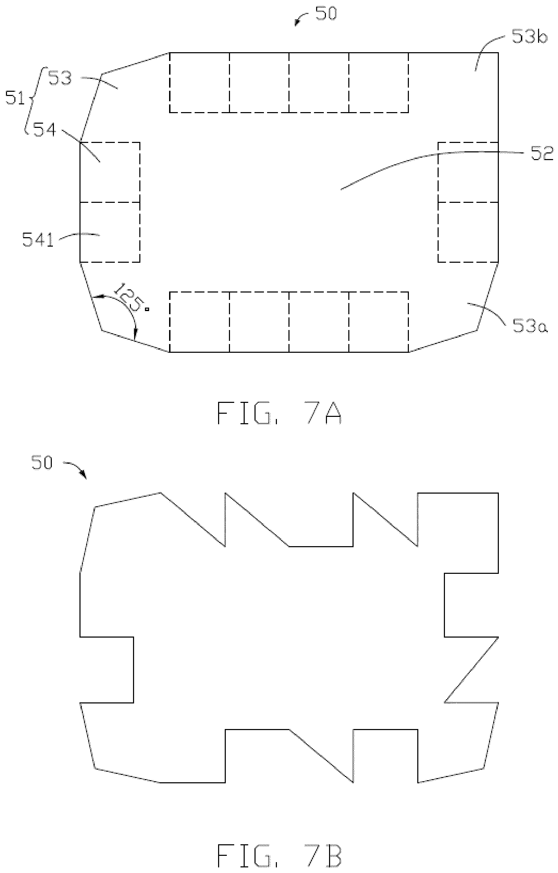

Referring to FIGS. 7A and 7B, which are diagrammatic views of a microbead before and after coding according to a fifth embodiment of the present disclosure. The microbead 50 is a non-circular microbead. Specifically, in the embodiment, the microbead 50 is a rectangular microbead, and the code is engraved on the outside of the microbead 50. Furthermore, the microbead 50 includes an edge region 51 and a central region 52 surrounded by the edge region 51. The edge region 51 includes corner regions 53 and side regions 54 besides the corner region 53. The corner regions 53 are where the four corners of the rectangle are located. The three corner regions 53a are set to have a shape different from that of the other corner region 53b. Specifically, in the embodiment, the three corner regions 53a are cut to have an obtuse-angle shape of approximately 125°. The other corner region 53b maintains a right-angle shape. It can be understood that setting the three corner regions 53a to be different from the other corner region 53b enables the computer identification device 900 to accurately position the microbead 50, so that the three corner regions 53a constitute the positioning device of the microbead 50. The side regions 54 are the regions where the long and short sides of the rectangle adjacent to the corner region 53 are located. At least one coding position 541 is provided on each side region 54, and the coding position 541 is used to engrave a code pattern. In the embodiment, the number of the coding positions 541 is 12 in total. The code pattern may be a rectangle, a triangle, or a combination thereof. FIG. 7B shows one code of the microbead 50. It can be understood that by engraving triangular code patterns or rectangular code patterns on different code positions 541, and then engraving a single triangular code pattern, a single rectangular code, or a combination thereof on different microbeads 50, the microbead 50 can obtain 3¹²=531441 different codes.

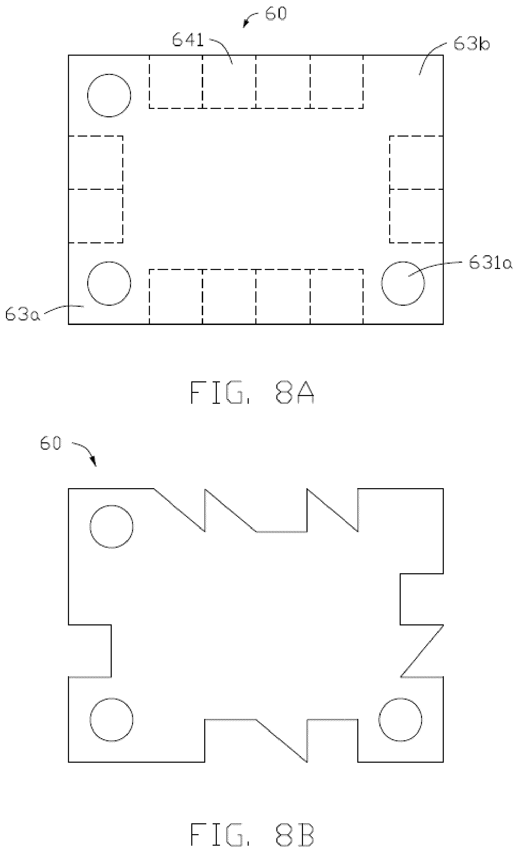

Referring to FIGS. 8A and 8B, which are diagrammatic views of a microbead before and after coding according to a sixth embodiment of the present disclosure. The microbead 60 in the embodiment is substantially the same as the microbead 50 in the fifth embodiment, except that three corner regions 63a in the embodiment are provided with positioning devices 631a, and the other corner region 63b is not provided with the positioning device. Specifically, the positioning device 631a may be a positioning hole or a positioning protrusion. The positioning device 631a may be located at the same position of the front and back sides of the microbead 60. In the embodiment shown in FIG. 8A and FIG. 8B, the positioning device 631a is a positioning hole penetrating through the front and back sides of the microbead 60, so that the same position of the front and back sides of the microbead 60 can be identified by the computer identification device 900. In other embodiments, the positioning device 631a may be a positioning protrusion located at a same position or different positions of the front and back sides of the microbead 60. The positioning device 631a may also be a positioning protrusion located on one side of the microbead 60 but not on the other side. By setting the positioning protrusion, when the microbead 60 is recognized by the computer identification device 900, no matter the front side or the back side of the microbead 60 faces the computer identification device 900, the computer identification device 900 can still recognize the predetermined starting position and direction of the code on the microbead 60 by the positioning device 631a. FIG. 8B shows one code of the microbead 60. It can be understood that by engraving triangular code patterns or rectangular code patterns on different code positions 641, and then engraving a single triangular code pattern, a single rectangular code, or a combination thereof on different microbeads 60, the microbeads 60 can obtain 3¹²=531441 different codes.

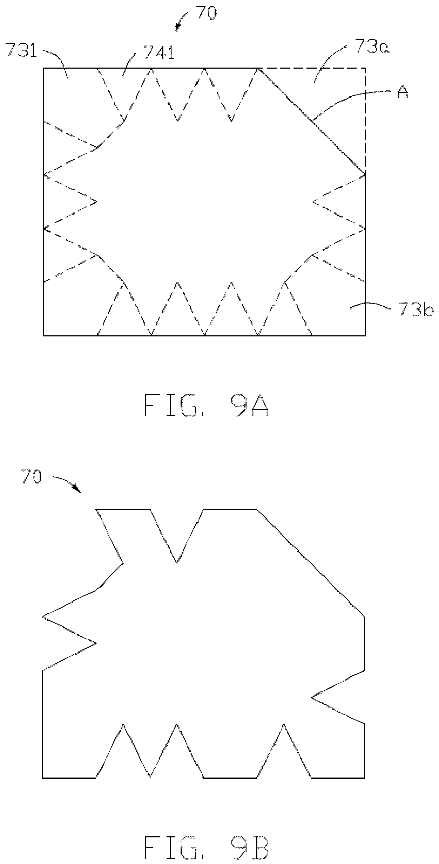

Referring to FIG. 9A and FIG. 9B, which are diagrammatic views of a microbead before and after coding according to a seventh embodiment of the present disclosure. The microbead 70 in the embodiment is substantially the same as the microbead 10 in the first embodiment. Difference is that except for the corner region 73a where the marking position A is located, the corner regions 73b of the microbead 70 in the embodiment also set the coding positions 731, thereby increasing the length of the code. FIG. 9B shows one code of the microbead 70. It can be understood that by engraving the code patterns on the different code positions 731 and 741, the microbead 70 can obtain more kinds of codes, which improves the utilization rate of the engravable region on the microbead 70.

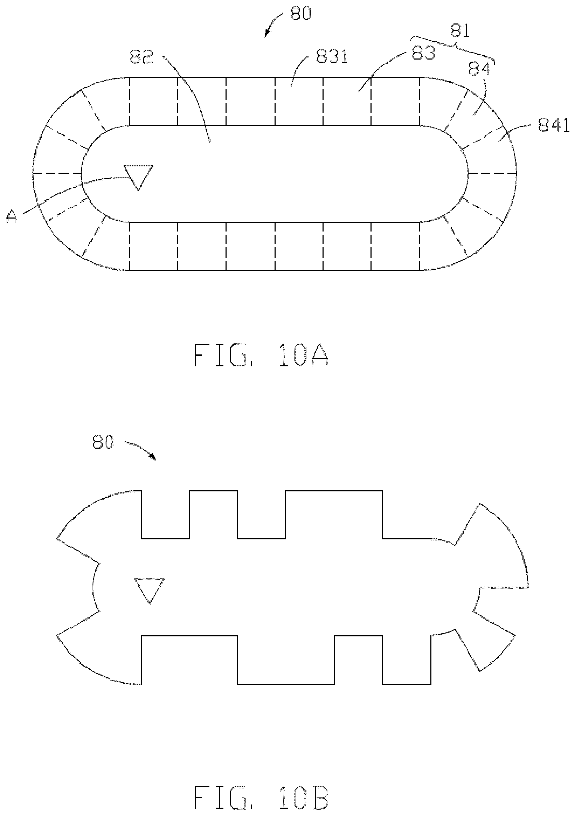

Referring to FIGS. 10A and 10B, which are diagrammatic views of a microbead before and after coding according to an eighth embodiment of the present disclosure which is not part of the invention. The microbead 80 is a non-circular microbead. Specifically, in the embodiment, the microbead 80 has an oblate shape, and the code is engraved on the outside of the microbeads 80. Furthermore, the microbead 80 includes an edge region 81 and a central region 82 surrounded by the edge region 81. The edge region 81 includes two straight side regions 83 and two arc side regions 84. Each of the two arc side regions 84 connects to two ends of the straight side regions 83 extending in the same direction, so that the entire edge region 81 has a surrounding structure surrounding the center region 82. A number of coding positions 831 and 841 are set on both the straight side regions 83 and the arc side regions 84. A marking position A is set on the central region 82, which deviates from the center of the central region 82. At the same time, the marking position A has a direction indicator, which is convenient for the computer identification device 900 to recognize the front and back sides of the microbead 80 and the starting position of the code according to the position and the direction indicator of the marking position A. In the embodiment, the marking position A is a triangular through hole or a triangular protrusion. When the triangular through hole is used as the marking position A, the triangular through hole penetrates the front and back surfaces of the microbead 80. When the triangular protrusion is used as the marking position A, the triangular protrusion needs to be set at the same position of the front and back sides of the microbead 80. In the embodiment, the corner of the triangular through hole or the triangular protrusion pointing to the side arc side region 84 can be used as the direction indicator.

FIG. 10B shows one code of the microbead 80. It can be understood that by engraving the code patterns on the different code positions 831 and 841, the microbead 80 can obtain a variety of different codes. Since the corner region is removed and replaced by the arc side regions 84 connecting to the straight side regions 83, the region of the microbead 80 that can be used to engrave the code is further expanded, so that the microbead 80 can obtain more different codes.

In other embodiments, the edge region of the microbead may also be the arc side regions, the straight side regions, and the corner regions connected in a direction surrounding the central region, and finally forming the entire edge region surrounding the central region. The positioning positions may be distributed on the arc side regions and the straight side regions, or on the corner regions at the same time.

In other embodiments, the edge region of the microbead may also be the arc side regions and the corner regions connected in a direction surrounding the central region, and finally forming the entire edge region surrounding the central region. The positioning positions may be distributed on the arc side regions or on the corner regions at the same time.

Referring to FIG. 11, which is a diagrammatic view of the microbead after coding according to a ninth embodiment of the present disclosure which is not part of the invention. The difference of the embodiment from the above embodiment is that the coding patterns on the microbead 90 also has a direction indicator, so as to prevent the computer identification device 900 to perform an error recognition due to breakage or shield of the marking position, which makes it impossible to recognize or even incorrectly recognize the direction of the code. Furthermore, since the coding patterns can indicate the direction, the positioning device or the marking position can be omitted. The computer identification device 900 can recognize the front and back sides of the microbead 90, the direction of the code, and the starting position of the code based on the direction of the coding pattern, or combined with other factors such as one of the positioning device and the marking position.

In a specific embodiment, the coding pattern has a preset vertex, which deviates to or deviates from a preset direction. The preset direction is preset as the direction of the code. The computer identification device 900 recognizes the deviated direction of the preset vertex, thereby recognizing the front and back sides of the microbead 90 and the direction of the code.

In the embodiment, the coding pattern is a triangular coding pattern, and the projection h of the vertex 910 of triangular coding pattern facing the microbead 90 projected on the bottom side opposite to the vertex 910 (shown by the dotted line in the figure) does not fall on the midpoint position i of the bottom side. The geometric center X of the coding pattern deviates from the geometric center O of the coding position. Thus, the triangular coding pattern as a whole presents a slanted state, and the vertex 910 is deviated to one side rather than being disposed at the center. The deviated direction of the vertex 910 is preset as the direction of the code of the microbead 90. After the computer identification device 900 recognizes the deviated direction of the preset vertex 910 of the triangular coding pattern, the front and back sides of the microbead 90, the direction of the code, and the starting position of the code can be recognized according to the marking position A.

It can be understood that in other embodiments, a coding pattern with the direction indicator can be set on a fixed coding position. The coding pattern indicates the front and back sides of the microbead, the direction of the code, and the starting position of the code through the direction indicator. Other coding patterns are distinguished from the above coding pattern with the direction indicator through the differences of shape, size, orientation, etc. At this time, the computer identification device 900 can recognize the front and back sides of the microbead, the direction of the code, and the starting position of the code by identifying the coding pattern with the direction indicator.

It can be understood that, in other embodiments, the directionality of the coding pattern is also used as a constituent factor of the coding. For example, when only the triangular coding patterns are used, each triangular coding pattern can be set to make the vertex to deviate to the left side or to the right side, thereby obtaining two coding combinations. It can be understood that the left deviation and the right deviation here are only used to describe different directions of the vertexes of the triangular coding pattern, and do not have further meaning to limit the present disclosure. Therefore, in different embodiments, the code of the microbead can be obtained by combining one or more constituent factors such as the shape, the size, and the direction of the coding patterns.

It can be understood that the microbeads are set to be rectangular or oblate in the above embodiments, and coding patterns are cut out on the periphery of the rectangular or oblate microbeads to form different codes. In other embodiments, the shapes of the microbeads can also be elliptical, trapezoidal, irregular, or other shapes deviating from circular. Since the shape of the microbead deviates from circular, compared to the circular microbead, a ratio of the overall surface area of the non-circular microbead with respect to the perimeter of the microbead before coding is smaller, and more coding positions can be set on the same consumable region, or the size of the consumable region can be reduced by setting a same number of coding positions. Thus, the utilization rate of microbeads can be improved. Furthermore, in some embodiments, as shown in the first, the second, the fourth, and the eighth embodiments described above for example, the microbead may have a first length in a first dimensional direction and a second length in a second dimensional direction perpendicular to the first dimensional direction. The first length is longer than the second length. The microbead further has a central region and an edge region surrounding the central region in the plane formed by the first dimensional direction and the second dimensional direction. The edge region is provided with coding positions for engraving the codes. Since the ratio of the first length to the second length increases, the ratio of the overall surface region of the microbead before coding to the perimeter of the microbead before coding can be further reduced, and the utilization rate of the microbead can be further improved.

Referring to FIG. 12, which is a diagrammatic view of a computer identification device 900 for identifying the code of the microbead. The computer identification device 900 includes an image capturing module 901 and a code identification module 902. The image capturing module 901 is used to capture an image of the microbead 1000. The code identification module 902 is used to identify the front and back sides of the microbead 1000, the starting position of the code, and the direction of the code based on the marking position of the microbead 1000, the positioning device, the direction indicator of the code patterns, or a combination thereof. The code identification module 902 is further used to identify the code of the microbead 1000 according to the coding pattern on each coding position. The microbead 1000 can be the microbead in the above embodiment or another microbead that have been appropriately modified but still fall within the scope of the disclosure. The image capturing module 901 may be a CMOS camera, a CCD camera, an infrared camera, or the like.

The code identification module 902 identifies the code of the microbead 1000 according to the code pattern on each coding position. Specifically, when the code on the microbead 1000 has only one code pattern, the code identification module 902 identifies whether the coding position is recessed or not to determine whether the coding position is engraved with the code pattern. When the code on the microbead 1000 has two or more code patterns, the code identification module 902 identifies the preset characteristics of each code pattern. For example, when the coding patterns have triangle or rectangle at the same time, in an embodiment, the coding identification module 902 first identifies whether one coding position is recessed, and when the coding position is recessed, then further identifies whether the code patten is triangle or rectangle according to a distance from a specific point of the edge of the coding position to another specific point inside the recess. The above specific point is predefined and input into the computer identification device 900 as a preset parameter or preset condition.

It can be understood that the computer identification device 900 may also include a suitable processing unit and a storage unit to complete the microbead code identification function required by the computer identification device 900.

It can be understood that the processing unit of the computer identification device 900 can execute a software program. The storage unit stores the software program executed by the processing unit, and can simultaneously store result data obtained by the processing unit executing the software program, so as to realize the microbead code identification function. The code identification module 902 appearing above is a summary and a description of the microbead code identification function realized by the computer identification device 900 executing the software program.

Even though information and advantages of the present embodiments have been set forth in the foregoing description, together with details of the structures and functions of the present embodiments, the disclosure is illustrative only. Changes may be made in detail, especially in matters of shape, size, and arrangement of parts within the principles of the present exemplary embodiments, to the full extent indicated by the plain meaning of the terms in which the appended claims are expressed.

## Claims

1. A microbead (10, 20, 30, 40a, 40b, 50, 60, 70, 80, 90) with a code engraved on an outside of the microbead (10, 20, 30, 40a, 40b, 50, 60, 70, 80, 90), the microbead (10, 20, 30, 40a, 40b, 50, 60, 70, 80, 90) comprising:
a central region (12, 52, 82); and
an edge region (11, 51, 81) surrounding the central region (12, 52, 82), an outer contour of the edge region (11, 51, 81) before and after engraving the code is non-circular, the edge region (11, 51, 81) comprising a plurality of coding positions (141, 241, 341, 441, 541, 641, 731, 741, 831, 841), the code of the microbead (10, 20, 30, 40a, 40b, 50, 60, 70, 80, 90) is engraved on the plurality of coding positions (141, 241, 341, 441, 541, 641, 731, 741, 831, 841), and each bit of the code corresponds to each of the plurality of coding positions (141, 241, 341, 441, 541, 641, 731, 741, 831, 841);
the outer contour of the edge region (11, 51, 81) is rectangular, the edge region (11, 51, 81) comprises corner regions (13, 13a, 13b, 23a, 33a, 33b, 43a, 53, 53a, 53b, 63a, 63b, 73a, 73b) and side regions (14, 54), the corner regions (13, 13a, 13b, 23a, 33a, 33b, 43a, 53, 53a, 53b, 63a, 63b, 73a, 73b) are where four corners of the rectangle are located, the side regions (14, 54) are where the long and short sides of the rectangle adjacent to the corner regions (13, 13a, 13b, 23a, 33a, 33b, 43a, 53, 53a, 53b, 63a, 63b, 73a, 73b) are located, and the corner regions (13, 13a, 13b, 23a, 33a, 33b, 43a, 53, 53a, 53b, 63a, 63b, 73a, 73b) and the side regions (14, 54) are connected to each other in a direction surrounding the central region (12, 52, 82);
**characterized in that**, the corner regions (13a, 13b, 23a, 33a, 33b, 43a, 53a, 53b, 63a, 63b, 73a, 73b) are provided with positioning devices and/or marking positions (A), and the positioning devices and/or the marking positions (A) are configured to allow a computer identification device (900) to identify a front side and a back side of the microbead (10, 20, 30, 40a, 40b, 50, 60, 70, 90), a starting position of the code, and a direction of the code.

2. The microbead (10, 20, 30, 40a, 40b, 50, 60, 70, 80, 90) of claim 1, **characterized in that**, the microbead (10, 20, 30, 40a, 40b, 50, 60, 70, 80, 90) has a first length in a first dimensional direction and a second length in a second dimensional direction perpendicular to the first dimensional direction, the first length is longer than the second length, and the edge region (11, 51, 81) surrounds the central region (12, 52, 82) at least on a plane formed by the first dimensional direction and the second dimensional direction.

3. The microbead (10, 20, 30, 40a, 40b, 50, 60, 70, 80, 90) of claim 2, **characterized in that**, the plurality of coding positions (141, 241, 341, 441, 541, 641, 731, 741, 831, 841) is at least arranged on the plane formed by the first dimensional direction and the second dimensional direction.

4. The microbead (50) of claim 1, **characterized in that**, a portion of the corner regions (53a) is processed to have a shape different from other portions of the corner regions (53b), and the portion of the corner region (53a) as a whole serves as the positioning device.

5. The microbead (60) of claim 1, **characterized in that**, a portion of the corner regions (63a) defines a through hole penetrating the microbead (60), and the through hole serves as the positioning device; or
a portion of the corner regions (63a) is provided with a positioning protrusion, and the positioning protrusion serves as the positioning device.

6. The microbead (10, 20, 30, 40a, 40b, 70) of claim 1, **characterized in that**, the marking position (A) is an oblique line segment or an arc segment generated by cutting one of the corner regions (13a, 23a, 33a, 43a, 73a).

7. The microbead (30) of claim 6, **characterized in that**, a center of a circle where the arc segment is located is on the microbead (30).

8. The microbead (10, 20, 30, 40a, 40b, 50, 60, 70) of claim 1, **characterized in that**, the plurality of coding positions (141, 241, 341, 441, 541, 641) is disposed on the side regions (14, 54); or
the plurality of coding positions (731, 741) is disposed on the side regions and the corner regions (73b).

9. The microbead (10, 20, 30, 40a, 40b, 50, 60, 70, 80, 90) of claim 1, **characterized in that**, the code is presented by engraving code patterns on the plurality of coding positions (141, 241, 341, 441, 541, 641, 731, 741, 831, 841), and the code patterns is a combination of one or more patterns.

10. The microbead (10, 20, 30, 40a, 40b, 50, 60, 70, 80, 90) of claim 9, **characterized in that**, the coding patterns are triangular coding patterns, rectangular coding patterns, trapezoidal coding patterns, or any combination thereof.

11. The microbead (90) of claim 9, **characterized in that**, the coding pattern of at least one of the plurality of coding positions is used to indicate a front side and a back side of the microbead (90) and a direction of the code.

12. The microbead (90) of claim 11, **characterized in that**, the coding pattern of the at least one of the plurality of coding positions has a preset vertex (910), the preset vertex (910) deviates to or from a preset direction, and the preset direction is the direction of the code on the microbead (90).

## Patentansprüche

1. Eine Mikroperle (10, 20, 30, 40a, 40b, 50, 60, 70, 80, 90) mit einem auf einer Außenseite der Mikroperle (10, 20, 30, 40a, 40b, 50, 60, 70, 80, 90) eingravierten Code, wobei die Mikroperle (10, 20, 30, 40a, 40b, 50, 60, 70, 80, 90) umfasst:
eine Zentralregion (12, 52, 82); und
eine Randregion (11, 51, 81), die die Zentralregion (12, 52, 82) umgibt, wobei ein äußerer Umriss der Randregion (11, 51, 81) vor und nach der Gravur des Codes nicht kreisförmig ist, wobei die Randregion (11, 51, 81) eine Mehrzahl von Codierpositionen (141, 241, 341, 441, 541, 641, 731, 741, 831, 841) umfasst, wobei der Code der Mikroperle (10, 20, 30, 40a, 40b, 50, 60, 70, 80, 90) auf der Mehrzahl von Codierpositionen (141, 241, 341, 441, 541, 641, 731, 741, 831, 841) eingraviert ist und jedes Bit des Codes jeweils einer der Mehrzahl von Codierpositionen (141, 241, 341, 441, 541, 641, 731, 741, 831, 841) entspricht;
wobei der äußere Umriss der Randregion (11, 51, 81) rechteckig ist, wobei die Randregion (11, 51, 81) Eckregionen (13, 13a, 13b, 23a, 33a, 33b, 43a, 53, 53a, 53b, 63a, 63b, 73a, 73b) und Seitenregionen (14, 54) umfasst, wobei sich die Eckregionen (13, 13a, 13b, 23a, 33a, 33b, 43a, 53, 53a, 53b, 63a, 63b, 73a, 73b) an den vier Ecken des Rechtecks befinden, wobei sich die Seitenregionen (14, 54) an den langen und kurzen Seiten des Rechtecks befinden, die an die Eckregionen (13, 13a, 13b, 23a, 33a, 33b, 43a, 53, 53a, 53b, 63a, 63b, 73a, 73b) angrenzen, und wobei die Eckregionen (13, 13a, 13b, 23a, 33a, 33b, 43a, 53, 53a, 53b, 63a, 63b, 73a, 73b) und die Seitenregionen (14, 54) miteinander in einer Richtung verbunden sind, die die Zentralregion (12, 52, 82) umgibt;
**dadurch gekennzeichnet, dass**
die Eckregionen (13a, 13b, 23a, 33a, 33b, 43a, 53a, 53b, 63a, 63b, 73a, 73b) mit Positionierungsvorrichtungen und/oder Markierungspositionen (A) versehen sind, wobei die Positionierungsvorrichtungen und/oder die Markierungspositionen (A) so ausgestaltet sind, dass sie eine Identifikation einer Vorderseite und einer Rückseite der Mikroperle (10, 20, 30, 40a, 40b, 50, 60, 70, 90), einer Startposition des Codes und einer Richtung des Codes durch eine Computer-Identifikationsvorrichtung (900) ermöglichen.

2. Die Mikroperle (10, 20, 30, 40a, 40b, 50, 60, 70, 80, 90) nach Anspruch 1, **dadurch gekennzeichnet, dass** die Mikroperle (10, 20, 30, 40a, 40b, 50, 60, 70, 80, 90) eine erste Länge in einer ersten Richtungsdimension und eine zweite Länge in einer zweiten Richtungsdimension aufweist, die senkrecht zur ersten Richtungsdimension verläuft, wobei die erste Länge größer ist als die zweite Länge, und wobei die Randregion (11, 51, 81) die Zentralregion (12, 52, 82) zumindest in einer Ebene umgibt, die durch die erste und die zweite Richtungsdimension gebildet ist.

3. Die Mikroperle (10, 20, 30, 40a, 40b, 50, 60, 70, 80, 90) nach Anspruch 2, **dadurch gekennzeichnet, dass** die Mehrzahl von Codierpositionen (141, 241, 341, 441, 541, 641, 731, 741, 831, 841) zumindest in der durch die erste und die zweite Richtungsdimension gebildeten Ebene angeordnet ist.

4. Die Mikroperle (50) nach Anspruch 1, **dadurch gekennzeichnet, dass** ein Teil der Eckregionen (53a) so ausgebildet ist, dass er sich in seiner Form von anderen Teilen der Eckregionen (53b) unterscheidet, und dass der genannte Teil der Eckregionen (53a) insgesamt als Positionierungsvorrichtung dient.

5. Die Mikroperle (60) nach Anspruch 1, **dadurch gekennzeichnet, dass** ein Teil der Eckregionen (63a) ein Durchgangsloch definiert, das die Mikroperle (60) durchdringt, wobei das Durchgangsloch als Positionierungsvorrichtung dient; oder
dass ein Teil der Eckregionen (63a) mit einer Positionierungswölbung versehen ist, wobei die Positionierungswölbung als Positionierungsvorrichtung dient.

6. Die Mikroperle (10, 20, 30, 40a, 40b, 70) nach Anspruch 1, **dadurch gekennzeichnet, dass** die Markierungsposition (A) ein schräger Linienabschnitt oder ein Kreisbogenabschnitt ist, der durch Ausschneiden einer der Eckregionen (13a, 23a, 33a, 43a, 73a) erzeugt ist.

7. Die Mikroperle (30) nach Anspruch 6, **dadurch gekennzeichnet, dass** sich ein Mittelpunkt des Kreises, auf dem sich der Kreisbogenabschnitt befindet, auf der Mikroperle (30) befindet.

8. Die Mikroperle (10, 20, 30, 40a, 40b, 50, 60, 70) nach Anspruch 1, **dadurch gekennzeichnet, dass** die Mehrzahl von Codierpositionen (141, 241, 341, 441, 541, 641) auf den Seitenregionen (14, 54) angeordnet ist; oder
dass die Mehrzahl von Codierpositionen (731, 741) auf den Seitenregionen und den Eckregionen (73b) angeordnet ist.

9. Die Mikroperle (10, 20, 30, 40a, 40b, 50, 60, 70, 80, 90) nach Anspruch 1, **dadurch gekennzeichnet, dass** der Code durch Gravur von Codiermustern auf die Mehrzahl von Codierpositionen (141, 241, 341, 441, 541, 641, 731, 741, 831, 841) dargestellt ist, wobei die Codiermuster eine Kombination aus einem oder mehreren Mustern sind.

10. Die Mikroperle (10, 20, 30, 40a, 40b, 50, 60, 70, 80, 90) nach Anspruch 9, **dadurch gekennzeichnet, dass** die Codiermuster dreieckige, rechteckige, trapezförmige Codiermuster oder eine beliebige Kombination davon sind.

11. Die Mikroperle (90) nach Anspruch 9, **dadurch gekennzeichnet, dass** das Codiermuster mindestens einer der Mehrzahl von Codierpositionen verwendet wird, um eine Vorderseite und eine Rückseite der Mikroperle (90) sowie eine Richtung des Codes anzuzeigen.

12. Die Mikroperle (90) nach Anspruch 11, **dadurch gekennzeichnet, dass** das Codiermuster der mindestens einen der Mehrzahl von Codierpositionen einen voreingestellten Scheitelpunkt (910) aufweist, wobei der voreingestellte Scheitelpunkt (910) zu oder von einer voreingestellten Richtung abweicht, und wobei die voreingestellte Richtung die Richtung des Codes auf der Mikroperle (90) ist.

## Revendications

1. Une microbille (10, 20, 30, 40a, 40b, 50, 60, 70, 80, 90) comportant un code gravé sur une surface extérieure de la microbille (10, 20, 30, 40a, 40b, 50, 60, 70, 80, 90), la microbille (10, 20, 30, 40a, 40b, 50, 60, 70, 80, 90) comprenant :
une région centrale (12, 52, 82) ; et
une région périphérique (11, 51, 81) entourant la région centrale (12, 52, 82), un contour extérieur de la région périphérique (11, 51, 81), avant et après la gravure du code, étant non circulaire, la région périphérique (11, 51, 81) comprenant une pluralité de positions de codage (141, 241, 341, 441, 541, 641, 731, 741, 831, 841), le code de la microbille (10, 20, 30, 40a, 40b, 50, 60, 70, 80, 90) étant gravé sur la pluralité de positions de codage (141, 241, 341, 441, 541, 641, 731, 741, 831, 841), et chaque bit du code correspondant à chacune desdites positions de codage (141, 241, 341, 441, 541, 641, 731, 741, 831, 841);
le contour extérieur de la région périphérique (11, 51, 81) étant rectangulaire, ladite région périphérique (11, 51, 81) comprenant des régions d'angle (13, 13a, 13b, 23a, 33a, 33b, 43a, 53, 53a, 53b, 63a, 63b, 73a, 73b) et des régions latérales (14, 54), lesdites régions d'angle (13, 13a, 13b, 23a, 33a, 33b, 43a, 53, 53a, 53b, 63a, 63b, 73a, 73b) étant situées aux quatre coins du rectangle, lesdites régions latérales (14, 54) étant situées au niveau des côtés longs et courts du rectangle adjacents auxdites régions d'angle (13, 13a, 13b, 23a, 33a, 33b, 43a, 53, 53a, 53b, 63a, 63b, 73a, 73b), et lesdites régions d'angle (13, 13a, 13b, 23a, 33a, 33b, 43a, 53, 53a, 53b, 63a, 63b, 73a, 73b) et lesdites régions latérales (14, 54) étant connectées entre elles dans une direction entourant la région centrale (12, 52, 82);
**caractérisée en ce que**
lesdites régions d'angle (13a, 13b, 23a, 33a, 33b, 43a, 53a, 53b, 63a, 63b, 73a, 73b) sont pourvues de dispositifs de positionnement et/ou de marques de repérage (A), lesdits dispositifs de positionnement et/ou lesdites marques de repérage (A) étant configurés pour permettre à un dispositif d'identification informatique (900) d'identifier une face avant et une face arrière de la microbille (10, 20, 30, 40a, 40b, 50, 60, 70, 90), une position de départ du code et une direction du code.

2. La microbille (10, 20, 30, 40a, 40b, 50, 60, 70, 80, 90) selon la revendication 1, **caractérisée en ce que** la microbille (10, 20, 30, 40a, 40b, 50, 60, 70, 80, 90) présente une première longueur dans une première direction dimensionnelle et une seconde longueur dans une seconde direction dimensionnelle perpendiculaire à la première direction dimensionnelle, la première longueur étant supérieure à la seconde longueur, et la région périphérique (11, 51, 81) entourant la région centrale (12, 52, 82) au moins sur un plan formé par la première et la seconde directions dimensionnelles.

3. La microbille (10, 20, 30, 40a, 40b, 50, 60, 70, 80, 90) selon la revendication 2, **caractérisée en ce que** la pluralité de positions de codage (141, 241, 341, 441, 541, 641, 731, 741, 831, 841) est au moins disposée sur le plan formé par la première et la seconde directions dimensionnelles.

4. La microbille (50) selon la revendication 1, **caractérisée en ce qu'**une partie des régions d'angle (53a) est façonnée de manière à avoir une forme différente des autres parties des régions d'angle (53b), et **en ce que** ladite partie des régions d'angle (53a) sert dans son ensemble de dispositif de positionnement.

5. La microbille (60) selon la revendication 1, **caractérisée en ce qu'**une partie des régions d'angle (63a) définit un trou traversant la microbille (60), ledit trou servant de dispositif de positionnement; ou
**en ce qu'**une partie des régions d'angle (63a) est pourvue d'une protubérance de positionnement, ladite protubérance servant de dispositif de positionnement.

6. La microbille (10, 20, 30, 40a, 40b, 70) selon la revendication 1, **caractérisée en ce que** la marque de repérage (A) est un segment de ligne oblique ou un segment d'arc généré par découpe de l'une des régions d'angle (13a, 23a, 33a, 43a, 73a).

7. La microbille (30) selon la revendication 6, **caractérisée en ce que** le centre du cercle sur lequel est situé le segment d'arc se trouve sur la microbille (30).

8. La microbille (10, 20, 30, 40a, 40b, 50, 60, 70) selon la revendication 1, **caractérisée en ce que** la pluralité de positions de codage (141, 241, 341, 441, 541, 641) est disposée sur les régions latérales (14, 54); ou
**en ce que** la pluralité de positions de codage (731, 741) est disposée sur les régions latérales et sur les régions d'angle (73b).

9. La microbille (10, 20, 30, 40a, 40b, 50, 60, 70, 80, 90) selon la revendication 1, **caractérisée en ce que** le code est représenté par gravure de motifs de codage sur la pluralité de positions de codage (141, 241, 341, 441, 541, 641, 731, 741, 831, 841), lesdits motifs de codage étant une combinaison d'un ou de plusieurs types de motifs.

10. La microbille (10, 20, 30, 40a, 40b, 50, 60, 70, 80, 90) selon la revendication 9, **caractérisée en ce que** les motifs de codage sont des motifs de codage triangulaires, rectangulaires, trapézoïdaux, ou toute combinaison de ceux-ci.

11. La microbille (90) selon la revendication 9, **caractérisée en ce que** le motif de codage d'au moins une des positions de codage est utilisé pour indiquer une face avant et une face arrière de la microbille (90) ainsi qu'une direction du code.

12. La microbille (90) selon la revendication 11, **caractérisée en ce que** le motif de codage d'au moins une des positions de codage présente un sommet prédéfini (910), ledit sommet prédéfini (910) s'écartant vers ou depuis une direction prédéfinie, ladite direction prédéfinie étant la direction du code sur la microbille (90).
